# EUROPEAN PATENT APPLICATION

(11) **EP 2 674 108 A1**
(43) Date of publication of application: **18.12.2013**
(21) Application number: 13170769.7
(22) Date of filing: 06.06.2013
(51) Int. Cl.: A61B 8/08, A61B 8/00

(54) **Ultrasound diagnosis method and apparatus using electrocardiogram**

(30) Priority: 11.06.2012 KR 20120062350
(71) Applicant: Samsung Medison Co., Ltd., Gangwon-do 250-870 (KR)
(72) Inventor: Lee, Jin-yong, Gangwon-do (KR); Lee, Bong-heon, Gangwon-do (KR); Chang, Eun-jung, Gangwon-do (KR)
(74) Representative: Schmid, Wolfgang

(57) **Abstract**

An ultrasound diagnosis method using an electrocardiogram (ECG) including: obtaining ECG information; generating first volume data using at least one piece of sub volume data obtained in a first ECG cycle based on the ECG information; generating second volume data using at least one piece of sub volume data obtained in a second ECG cycle based on the ECG information; and displaying at least one of the first volume data and the second volume data.

## Description

### CROSS-REFERENCE TO RELATED PATENT APPLICATION

This application claims the benefit of Korean Patent Application No. 10-2012-0062350, filed on June 11, 2012, in the Korean Intellectual Property Office, the disclosure of which is incorporated herein in its entirety by reference.

### BACKGROUND OF THE INVENTION

### 1. Field of the Invention

The present invention relates to a medical technology, and more particularly to an ultrasound diagnosis.

### 2. Description of the Related Art

Ultrasound diagnosis apparatuses generate an ultrasound signal (generally greater than 20 kHz) regarding a predetermined part inside a target body by using a probe, and obtain an image of the part inside the target body by using information regarding a reflected echo signal. In particular, ultrasound diagnosis apparatuses are used for medical purposes, such as detection of impurities inside the target body, measurement and observation of a wound thereof, etc. Such ultrasound diagnosis apparatuses have various advantages, including real-time display and safety because there is no radioactive exposure compared to X-ray apparatuses, and thus, the ultrasound diagnosis apparatuses are commonly used together with other image diagnosis apparatuses.

An image (hereinafter referred to as an ultrasound image) obtained through ultrasound diagnosis apparatuses may be displayed on the ultrasound diagnosis apparatuses or may be stored in a storage medium and displayed on other ultrasound diagnosis apparatuses. For example, the ultrasound image may be reduced and displayed on screens of cellular phones, portable electronic devices, personal digital assistant (PDAs), or tablet PCs, etc.

A sinoatrial (SA) node in a heart of a body generates an electrical signal to regulate a heartbeat. The electrical signal is transferred to the whole heart according to an electrical conductive system in the heart.

An electrocardiogram (ECG) measures the electrical signal generated by the SA node of the heart to regulate heartbeat and records the measured electrical signal in a graph. Such a process is referred to as electrocardiography. Types of the electrocardiography include a 12 lead ECG, an exercise stress ECG, an ambulatory Holter ECG, etc. A result of the electrocardiography is generally expressed in the graph regarding time and voltage sizes. Sizes and shapes of waveforms indicating results of measurement of each part of the body may be analyzed to determine whether the heartbeat is regular and to determine a rate of the heartbeat.

### SUMMARY OF THE INVENTION

The present invention provides a method and apparatus for obtaining high resolution 3D volume data using a plurality of pieces of sub volume data. The 3D volume data is obtained using an electrocardiogram (ECG), and thus a target body may be diagnosed in detail through each piece of the sub volume data.

According to an aspect of the present invention, there is provided an ultrasound diagnosis method using an electrocardiogram (ECG), the method including: obtaining ECG information; generating first volume data using at least one piece of sub volume data obtained in a first ECG cycle based on the ECG information; generating second volume data using at least one piece of sub volume data obtained in a second ECG cycle based on the ECG information; and displaying at least one of the first volume data and the second volume data.

The ultrasound diagnosis method may further include: determining an ECG cycle based on a time interval of ECG signals included in the ECG information.

In a case where the time interval of the ECG signals is within a previously determined range, the ECG cycle may be determined as the first ECG cycle; and in a case where the time interval of the ECG signals is outside the previously determined range, the ECG cycle may be determined as the second ECG cycle.

The first ECG cycle may be a normal ECG cycle, and the second ECG cycle may be an abnormal ECG cycle.

The first volume data may be normal volume data based on the normal ECG cycle, and the second volume data may be abnormal volume data based on the abnormal ECG cycle.

The ultrasound diagnosis method may further include: splitting a 3D scan space into a plurality of sub scan spaces; and obtaining a plurality of pieces of sub volume data corresponding to the plurality of respective sub scan spaces by sequentially scanning the plurality of sub scan spaces according to the ECG cycles included in the ECG information.

The ultrasound diagnosis method may further include: splitting a 3D scan space into a plurality of sub scan spaces; and obtaining a plurality of pieces of sub volume data corresponding to the sub scan spaces selected from the plurality of sub scan spaces based on an external input signal.

The ultrasound diagnosis method may further include: splitting a 3D scan space into a plurality of sub scan spaces, wherein the displaying includes: displaying volume data including all of a plurality of pieces of sub volume data corresponding to the plurality of respective sub scan spaces.

The displaying may include: displaying at least one of the at least one sub volume data used to generate the first volume data and the at least one sub volume data used to generate the second volume data and an identification mark.

The abnormal ECG cycle may include at least one of an atrial flutter ECG cycle, an atrial fibrillation ECG cycle, a ventricular flutter ECG cycle, and a ventricular fibrillation ECG cycle.

According to another aspect of the present invention, there is provided an ultrasound diagnosis apparatus using an ECG, the apparatus including: an ECG information obtaining unit for obtaining ECG information; a volume data processing unit for generating first volume data using at least one piece of sub volume data obtained in a first ECG cycle based on the ECG information, and generating second volume data using at least one piece of sub volume data obtained in a second ECG cycle based on the ECG information; and a display unit for displaying at least one of the first volume data and the second volume data.

According to another aspect of the present invention, there is provided a computer readable recording medium having embodied thereon a computer program for executing the ultrasound diagnosis method using an ECG.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of an ultrasound diagnosis apparatus according to an embodiment of the present invention;
FIG. 2 is a flowchart illustrating an ultrasound diagnosis method according to an embodiment of the present invention;
FIG. 3 is a flowchart illustrating an ultrasound diagnosis method according to another embodiment of the present invention;
FIGS. 4A and 4B show an embodiment in which a 3D scan space is split into a plurality of sub scan spaces;
FIG. 5 shows an embodiment in which volume data is generated using sub volume data based on ECG information;
FIG. 6 shows an embodiment in which first volume data and second volume data are generated using sub volume data based on ECG information;
FIG. 7 shows an embodiment in which a plurality of pieces of sub volume data is obtained based on an external input signal; and
FIG. 8 shows an embodiment in which at least one of first volume data and second volume data is displayed.

### DETAILED DESCRIPTION OF THE INVENTION

Most of the terms used herein are general terms that have been widely used in the technical art to which the present invention pertains. However, some of the terms used herein may be created reflecting intentions of technicians in this art, precedents, or new technologies. Also, some of the terms used herein may be arbitrarily chosen by the present applicant. In this case, these terms are defined in detail below. Accordingly, the specific terms used herein should be understood based on the unique meanings thereof and the whole context of the present invention.

In the present specification, it should be understood that the terms, such as 'including' or 'having,' etc., are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added. Also, the terms, such as 'unit' or 'module', etc., should be understood as a unit that processes at least one function or operation and that may be embodied in a hardware manner, a software manner, or a combination of the hardware manner and the software manner. As used herein, expressions such as "at least one of," when preceding a list of elements, modify the entire list of elements and do not modify the individual elements of the list.

The embodiments of the present invention will now be described more fully with reference to the accompanying drawings.

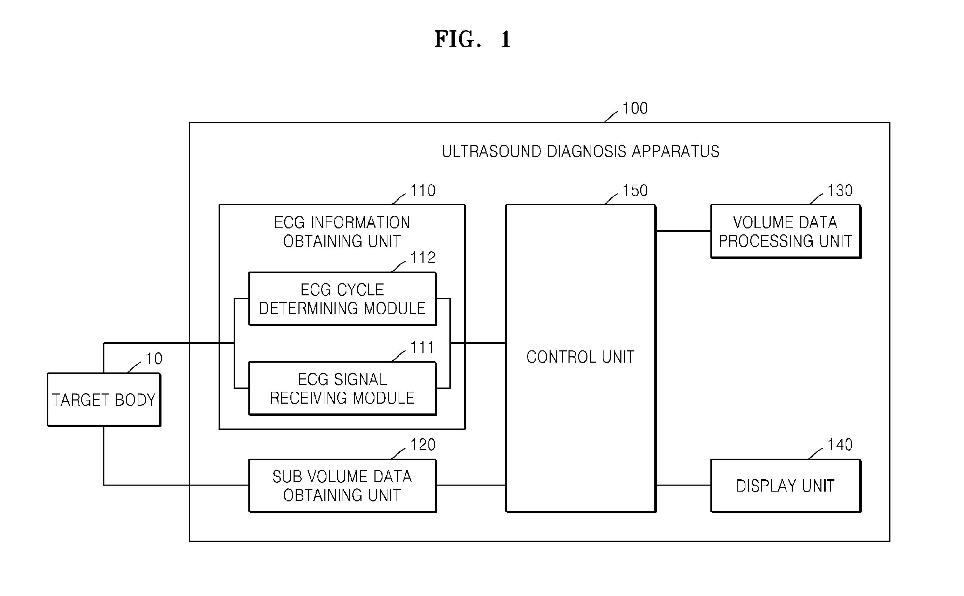

FIG. 1 is a block diagram of an ultrasound diagnosis apparatus 100 according to an embodiment of the present invention.

Referring to FIG. 1, the ultrasound diagnosis apparatus 100 according to an embodiment of the present invention may include an electrocardiogram (ECG) information obtaining unit 110, a sub volume data obtaining unit 120, a volume data processing unit 130, a display unit 140, and a control unit 150.

The ECG information obtaining unit 110 obtains ECG information measured from a target body 10 according to electrocardiography. The ECG information may include information regarding a cycle and intensity of an ECG signal measured from the target body 10. Also, the ECG information may include information regarding whether the cycle of the ECG signal is a normal ECG cycle within a previously determined range or an abnormal ECG cycle outside the previously determined range.

The ECG information may be obtained through electrical signals generated in a plurality of parts of the target body 10. The ECG information may be received through various parts of the target body 10 according to types of electrocardiography. For example, in the case of a 12 lead ECG, the ECG information obtaining unit 110 may obtain the ECG information through both wrists, both ankles, and a heart of the target body 10.

The ECG information obtaining unit 110 may include an ECG signal receiving module 111 and an ECG cycle determining module 112. The ECG signal receiving module 111 may receive a plurality of ECG signals from the target body 10. The received ECG signals may be electrical signals. Also, the ECG signal receiving module 111 may physically contact the target body 10 and receive electrical signals from the plurality of parts. The ECG signal receiving module 111 may measure variations of voltages induced through a plurality of electrodes.

The ECG information received by the ECG information obtaining unit 110 may include the plurality of ECG signals. Each ECG signal may be received at regular or irregular times according to a heartbeat. Hereinafter, the ECG cycle is referred to as a time interval between one of the plurality of ECG signals included in the ECG information and a subsequent ECG signal.

The ECG cycle determining module 112 may determine ECG cycles of the plurality of ECG signals based on the ECG information received from the ECG signal receiving module 111. In a case where the cycle of the ECG signal is within the previously determined range, the ECG cycle determining module 112 may determine the ECG cycle as a first ECG cycle. In a case where the cycle of the ECG signal is outside the previously determined range, the ECG cycle determining module 112 may determine the ECG cycle as a second ECG cycle.

According to an embodiment, the first ECG cycle may be a normal ECG cycle, and the second ECG cycle may be an abnormal ECG cycle. That is, the ECG cycle determining module 112 may determine the ECG cycle within the previously determined range as the normal ECG cycle, and determine the ECG cycle outside the previously determined range as the abnormal ECG cycle.

For example, in a case where the target body 10 is a human body, a general heartbeat rate of the human body is between 60 and 100. That is, the time interval of the ECG signal may be between 0.6 and 1 second (60/100 seconds and 60/60 seconds). Accordingly, in a case where the time interval of the ECG signal may be between 0.6 and 1 second, the ECG cycle determining module 112 may determine a cycle of the corresponding ECG signal as the first ECG cycle, and, in a case where the time interval of the ECG signal may be outside a range of between 0.6 and 1 second, the ECG cycle determining module 112 may determine a cycle of the corresponding ECG signal as the second ECG cycle. The above-described heartbeat rate is merely exemplary, and may vary with respect to an age of the target body 10, a health condition thereof, etc. Also, the previously determined range of the ECG cycle may be set by a user of the ultrasound diagnosis apparatus 100.

According to another embodiment, the abnormal ECG cycle may include at least one of an atrial flutter ECG cycle, an atrial fibrillation ECG cycle, a ventricular flutter ECG cycle, and a ventricular fibrillation ECG cycle. That is, the abnormal ECG cycle may mean an ECG cycle in a case where the heartbeat of the target body 10 is abnormally fast or slow according to an arrhythmia. The above description regarding the abnormal ECG cycle is merely an example. It will be understood by those of ordinary skill in the art that there may be various descriptions regarding the abnormal ECG cycle besides the above description.

The ECG information obtaining unit 110 may transmit the ECG information received by the ECG signal receiving module 111 and the ECG cycle determined by the ECG cycle determining module 112 to the control unit 150.

The sub volume data obtaining unit 120 may scan the target body 10 and obtain ultrasound data. The ultrasound data obtained by the sub volume data obtaining unit 120 may be 2D data or 3D data. The sub volume data obtaining unit 120 may be a probe that scans the target body 10, for example, a 2D probe or a 3D probe, etc.

The sub volume data obtaining unit 120 may obtain a plurality of pieces of sub volume data from the target body 10. The sub volume data means partial volume data constituting the 3D volume data. That is, the ultrasound diagnosis apparatus 100 may obtain the 3D volume data using the plurality of pieces of sub volume data obtained by the sub volume data obtaining unit 120.

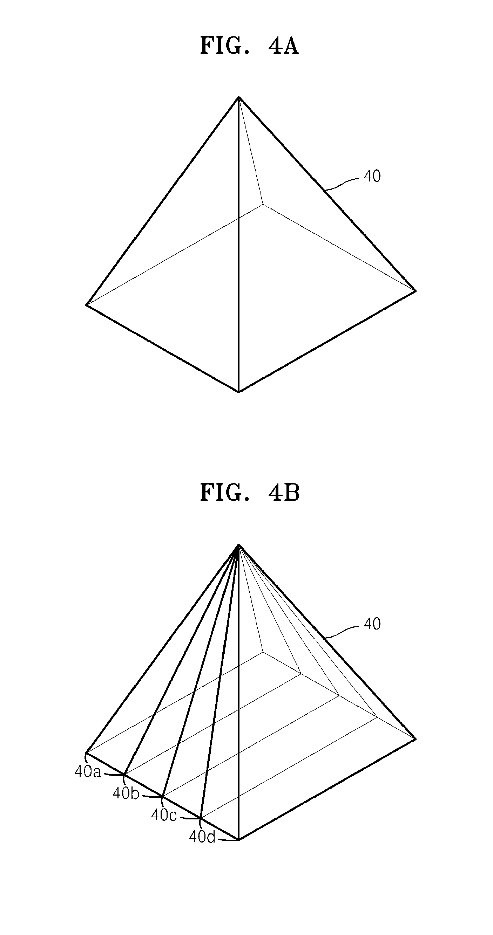

Also, the sub volume data obtaining unit 120 may obtain the sub volume data through a plurality of sub scan spaces that split from a 3D scan space. The 3D scan space and the sub scan spaces will be described in more detail with reference to FIG. 4.

According to an embodiment, the sub volume data obtaining unit 120 may obtain the plurality of pieces of sub volume data using the ECG information obtained by the ECG information obtaining unit 110. That is, the sub volume data obtaining unit 120 may obtain the plurality of pieces of sub volume data according to the ECG signals included in the ECG information.

For example, in a case where the ECG information obtaining unit 110 obtains electrical signals with respect to the heartbeat, the sub volume data obtaining unit 120 may scan the target body 10 and obtain first sub volume data until a subsequent electrical signal is received. Thereafter, if the subsequent electrical signal is received, the sub volume data obtaining unit 120 may obtain second sub volume data. The ECG information obtaining unit 110 may scan the target body 10 along the 3D scan space.

According to another embodiment, the ECG information obtaining unit 110 may obtain the sub volume data based on an external input signal received through a user input unit (not shown). The obtaining of the sub volume data in the ECG information obtaining unit 110 will be described in more detail with reference to FIGS. 4 through 7.

The volume data processing unit 130 may generate the 3D volume data using at least one piece of sub volume data obtained by the sub volume data obtaining unit 120. That is, the volume data processing unit 130 may generate the complete 3D volume data by combining at least one piece of sub volume data. For example, the volume data processing unit 130 may generate one piece of the complete 3D volume data by continuously connecting four pieces of sub volume data.

According to an embodiment, the volume data processing unit 130 may generate the 3D volume data by sequentially connecting at least one piece of sub volume data. The sequentially connected at least one piece of sub volume data may be sub volume data sequentially obtained through the plurality of sub scan spaces that are split from the 3D scan space.

According to another embodiment, the volume data processing unit 130 may generate the 3D volume data by using the sub volume data selected by the external input signal. For example, in a case where four pieces of sub volume data constitutes the 3D volume data, the sub volume data obtaining unit 120 may newly obtain third sub volume data selected by the external input signal, and the volume data processing unit 130 may generate the 3D volume data again by replacing previous third sub volume data with new third sub volume data.

The volume data processing unit 130 may generate first volume data and second volume data based on ECG cycles of the sub volume data. That is, the volume data processing unit 130 may generate the first volume data using at least one piece of sub volume data obtained in the first ECG cycle and the second volume data using at least one piece of sub volume data obtained in the second ECG cycle. The first volume data may be normal volume data. The second volume data may be abnormal volume data.

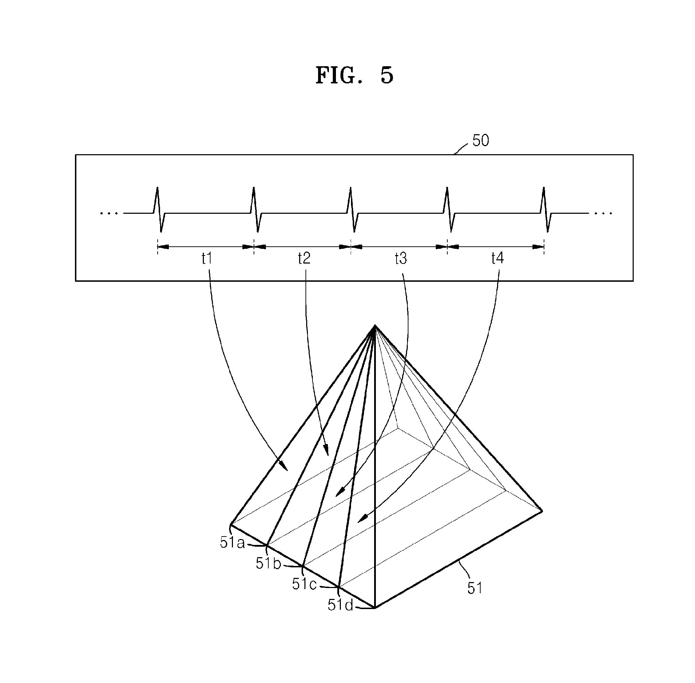

Also, the volume data processing unit 130 may generate the volume data using the whole sub volume data corresponding to each of the plurality of sub scan spaces. Alternatively, although the sub volume data corresponding to each of the plurality of sub scan spaces is not wholly obtained, the volume data processing unit 130 may generate the volume data by only using obtained sub volume data. A process of generating the volume data in the volume data processing unit 130 will be described in more detail with reference to FIGS. 5 through 7.

The display unit 140 may display at least one of the first volume data and the second volume data generated by the volume data processing unit 130. Also, according to an embodiment, the display unit 140 may display at least one of the plurality of sub volume data and an identification mark according to the control signal received from the control unit 150. That is, the display unit 140 may use the identification mark to visually distinguish selected sub volume data from other pieces of volume data. The identification mark may be, for example, a method of changing at least one of a color, a chroma, and brightness of the sub volume data or a method of displaying borders of the sub volume data in thick lines or displaying the sub volume data with markers.

The display unit 140 may include at least one of a liquid crystal display (LCD), a thin film transistor liquid crystal display (TFT-LCD), an organic light emitting diode (OLED), a flexible display, and a 3D display. Also, the ultrasound diagnosis apparatus 100 may include two or more display units 140 according to an implementation shape thereof.

According to an embodiment, the display unit 140 may be a touch screen that includes the above-described user input unit (not shown) and a layer structure. That is, the display unit 140 may be used as both an output apparatus and an input apparatus. In this case, the display unit 140 may receive a touch input using a stylus pen or a part of the human body.

Also, according to another embodiment, the display unit 140 may display the ECG information received from the ECG information obtaining unit 110. That is, the display unit 140 may express and display the plurality of ECG signals in a graph regarding time and intensity of voltages.

The control unit 150 may generally control the ECG information obtaining unit 110, the sub volume data obtaining unit 120, the volume data processing unit 130, and the display unit 140. More specifically, the control unit 150 may control the sub volume data obtaining unit 120 to obtain the sub volume data according to the ECG signals based on the ECG information received from the ECG information obtaining unit 110. That is, the control unit 150 may control the sub volume data obtaining unit 120 to sequentially change the sub scan spaces, and obtain the sub volume data whenever the sub volume data obtaining unit 120 measures the heartbeat.

Also, the control unit 150 may split the 3D scan space into the plurality of sub scan spaces. That is, the control unit 150 may control the sub volume data obtaining unit 120 to obtain the plurality of pieces of connected sub volume data through the split sub scan spaces. Alternatively, the control unit 150 may control the sub volume data obtaining unit 120 to continuously obtain the sub volume data from the sub volume data selected by the external input signal. The 3D scan space and the sub scan spaces will be described again later with reference to FIG. 4.

According to another embodiment, the control unit 150 may control the volume data processing unit 130 to generate the volume data according to the ECG cycles determined by the ECG information obtaining unit 110. That is, the control unit 150 may receive information regarding the ECG cycles determined by the ECG information obtaining unit 110, receive information regarding the sub volume data obtained by the sub volume data obtaining unit 120, and transmit the received information as the volume data. Thereafter, the control unit 150 may control the volume data processing unit 130 to generate the normal volume data or the abnormal volume data according to the ECG cycles corresponding to the sub volume data.

Also, the control unit 150 may control the display unit 140 to display volume data including the whole sub volume data corresponding to the plurality of sub scan spaces. Hereinafter, the volume data including the whole sub volume data corresponding to the plurality of sub scan spaces is referred to as "complete" volume data.

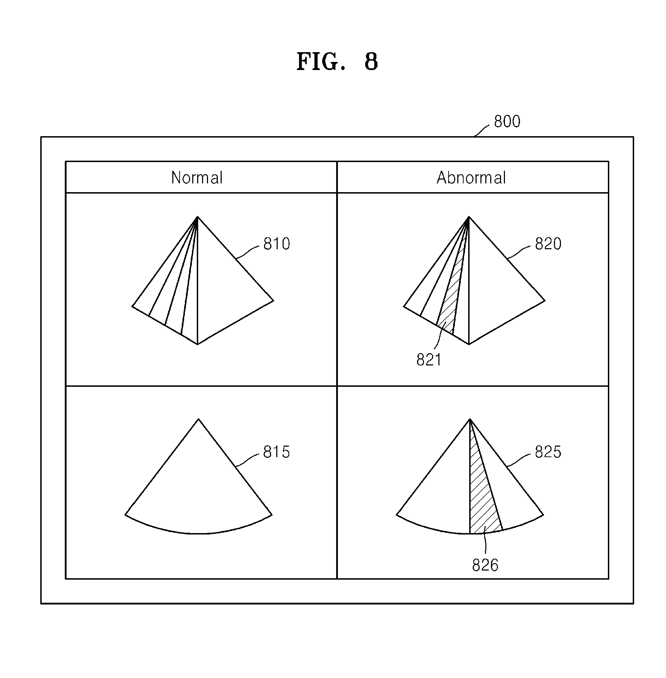

That is, the control unit 150 may control the display unit 140 to display the "complete" volume data among the first volume data and the second volume data. Accordingly, the display unit 140 may display at least one of the first volume data and the second volume data. According to another embodiment, the control unit 150 may control the display unit 140 to display volume data not including the whole sub volume data corresponding to the plurality of sub scan spaces, i.e., "incomplete" volume data. This will be described in detail later with reference to FIG. 8.

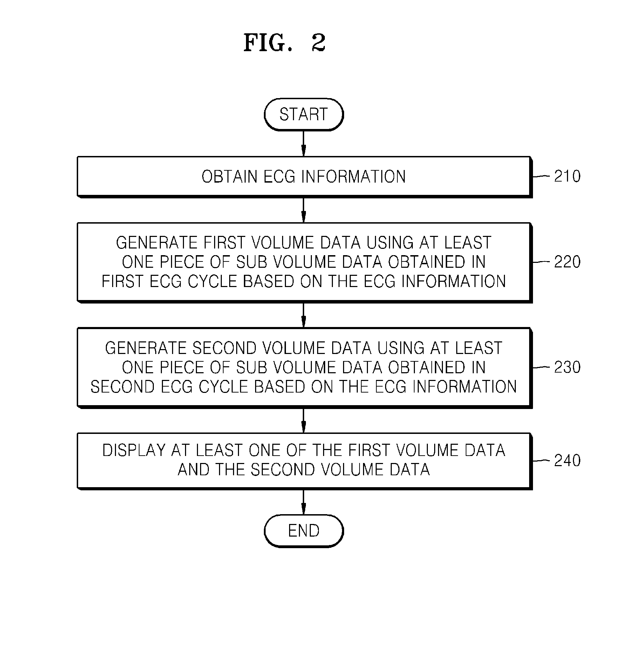

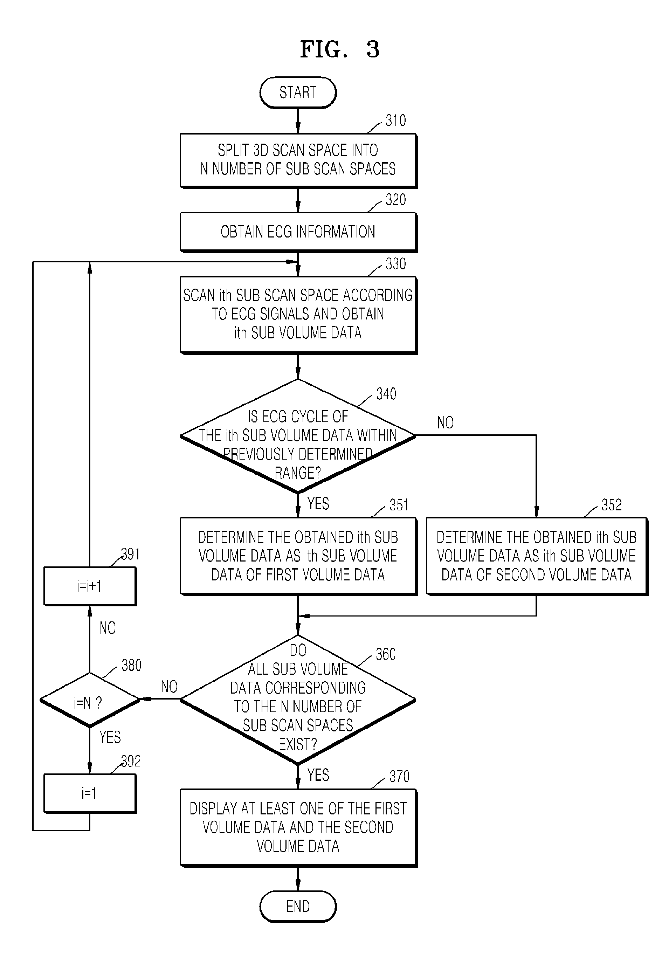

FIGS. 2 and 3 are flowcharts illustrating an ultrasound diagnosis method according to embodiments of the present invention. The methods of FIGS. 2 and 3 include operations time-sequentially performed by the ultrasound diagnosis apparatus 100, the ECG information obtaining unit 110, the sub volume data obtaining unit 120, the volume data processing unit 130, the display unit 140, and the control unit 150 of FIG. 1. Thus, although omitted below, the descriptions of the elements of FIG. 1 may also apply to the methods of FIGS. 2 and 3.

Hereinafter, an ultrasound diagnosis method using an ECG by using the construction of the ultrasound diagnosis apparatus 100 will now be described with reference to FIG. 2.

In operation 210, ECG information is obtained. The ECG information may include a plurality of ECG signals that may be continuously obtained at a time. As such, a time interval between the ECG signals is an ECG cycle as described above.

ECG cycles of the plurality of ECG signals included in the ECG information may be determined as first ECG cycles (normal ECG cycles) or second ECG cycles (abnormal ECG cycles) according to whether the ECG cycles are within a previously determined range. The ECG information obtained in operation 210 may be used to obtain a plurality of pieces of sub volume data.

In operation 220, first volume data is generated using at least one piece of sub volume data obtained in the first ECG cycles based on the ECG information. The at least one piece of sub volume data may be obtained using the ECG information obtained in operation 210. The first volume data may be generated by connecting a plurality of pieces of sequential sub volume data. The first ECG cycles may be normal ECG cycles within the previously determined range and thus the first volume data may be normal volume data.

In operation 230, second volume data is generated using at least one piece of sub volume data obtained in the second ECG cycles based on the ECG information. The at least one piece of sub volume data may be obtained using the ECG information obtained in operation 210, like in operation 220. Also, a plurality of sub volume data may be sub volume data received based on an external input signal received from a user. Unlike operation 220, the second ECG cycles may be abnormal ECG cycles outside the previously determined range and thus the second volume data may be abnormal volume data.

In operation 240, at least one of the first volume data and the second volume data is displayed. As described in operation 220, the first volume data is volume data generated using the at least one piece of sub volume data obtained in the first ECG cycles. As described in operation 230, the second volume data is volume data generated using the at least one piece of sub volume data obtained in the second ECG cycles.

According to an embodiment, "complete" volume data among the first volume data and the second volume data may be displayed in operation 240. That is, volume data including the whole sub volume data corresponding to a plurality of sub scan spaces may be displayed. According to another embodiment, the complete volume data and "incomplete" volume data may be displayed together in operation 240. That is, although the whole sub volume data corresponding to the plurality of sub scan spaces is not obtained, the display unit 140 may display volume data (the incomplete volume data) generated only using obtained sub volume data.

According to another embodiment, the ECG information may be expressed in a graph and displayed along with at least one of the first volume data and the second volume data in operation 240.

Thereafter, the ultrasound diagnosis method using the ECG will now be described in detail with reference to an embodiment of the present invention of FIG. 3.

In operation 310, the ultrasound diagnosis apparatus 100 splits a 3D scan space into an N number of sub scan spaces (N=1, 2, 3, ...). Referring to an embodiment of FIG. 4, a 3D scan space 40 of FIG. 4A is split into four sub scan spaces 40a, 40b, 40c, and 40d of FIG. 4B.

As the number N of the sub scan spaces increases, a resolution of the volume data increases, whereas it takes a long time to obtain the whole sub volume data corresponding to the number N of the sub scan spaces. Thus, the number N of the split sub scan spaces may be determined based on at least one of performance of a probe included in the ultrasound diagnosis apparatus 100 and the resolution of the volume data to be displayed.

In other words, since the probe scans the sub scan spaces to obtain the sub volume data, the narrower the range of the sub scan spaces the same probe scans, the higher the resolution of the sub volume data that may be obtained. This is why the more the same 3D scan space splits into sub scan spaces, the narrower the ultrasound beam emitted by the probe is collimated. However, the more the number N increases, the longer it takes to sequentially change the sub scan spaces and obtain the sub volume data.

In operation 320, the ultrasound diagnosis apparatus 100 obtains the ECG information. As described in operation 210 of FIG. 2, the ECG information may include a plurality of ECG signals that may be continuously obtained according to ECG cycles that are time intervals between the ECG signals.

In operation 330, the ultrasound diagnosis apparatus 100 scans an ith sub scan space according to the ECG signals and obtains ith sub volume data (i=1, 2, 3, ..., N). The ith sub scan space may be a sub scan space selected from the N number of sub scan spaces split in operation 310. That is, referring to FIG. 4B, the ith sub scan space may be each of the first sub scan space 40a, the second sub scan space 40b, the third sub scan space 40c, and the fourth sub scan space 40d.

More specifically, referring to FIG. 5 with respect to operation 330, the ultrasound diagnosis apparatus 100 may scan a 3D sub scan space 51 that is split into four sub scan spaces 51a, 51b, 51c, and 51d and obtain four pieces of sub volume data according to the ECG cycles.

That is, a probe scans a first sub scan space 51a according to a first ECG signal and obtains first sub volume data during an ECG cycle t1. Thereafter, if a second ECG signal is measured, a second sub scan space 51b is scanned, and second sub volume data is obtained during an ECG cycle t2. Likewise, during subsequent ECG cycles t3 and t4, third sub volume data and fourth sub volume data are obtained.

In other words, the ith sub scan space scanned in operation 330 may sequentially scan the N number of sub scan spaces. This will be described in operations 380, 391, and 392 again.

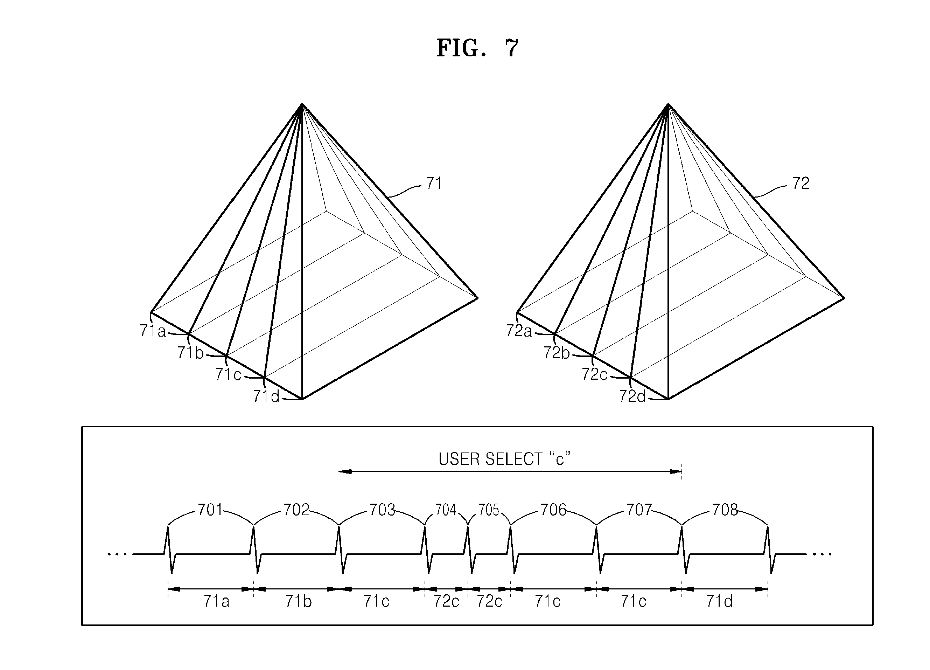

According to another embodiment, the ultrasound diagnosis apparatus 100 may scan the ith sub scan space determined by an external input signal in operation 330. That is, the scanned ith sub scan space may be determined based on a user input that selects one of the N number of sub scan spaces. The present embodiment will be described further with reference to FIG. 7.

In operation 340, the ultrasound diagnosis apparatus 100 determines whether an ECG cycle of the obtained ith sub volume data is within a previously determined range. The previously determined range of the ECG cycle may be determined according to a time interval between heartbeats based on a heartbeat rate per minute in a general case. Also, the previously determined range may be determined based on various factors such as an age of a target body, a sex thereof, a body condition, etc.

If the ultrasound diagnosis apparatus 100 determines that the ECG cycle of the obtained ith sub volume data is within the previously determined range in operation 340, the ultrasound diagnosis apparatus 100 performs operation 351, and, if the ultrasound diagnosis apparatus 100 determines that the ECG cycle of the obtained ith sub volume data is outside the previously determined range in operation 340, the ultrasound diagnosis apparatus 100 performs operation 352.

In operation 351, the ECG cycle of the obtained ith sub volume data is a first ECG cycle within the previously determined range, and thus the ultrasound diagnosis apparatus 100 determines the obtained ith sub volume data as ith sub volume data of the first volume data. That is, the ith sub volume data obtained in operation 330 may be determined as sub volume data used to generate the first volume data.

In operation 352, the ECG cycle of the obtained ith sub volume data is a second ECG cycle outside the previously determined range, and thus the ultrasound diagnosis apparatus 100 determines the obtained ith sub volume data as ith sub volume data of the second volume data. That is, the ith sub volume data obtained in operation 330 may be determined as sub volume data used to generate the second volume data.

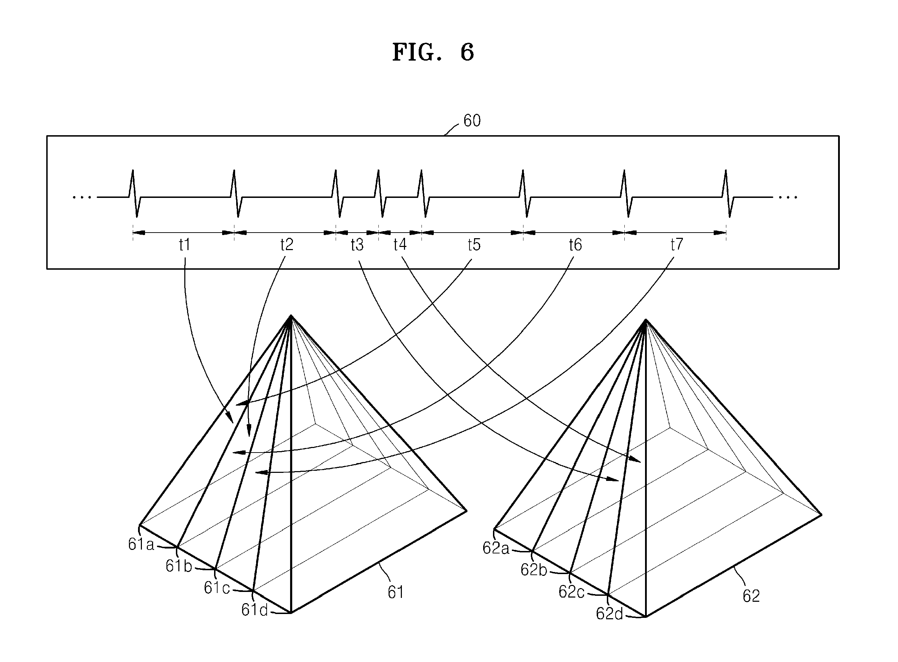

Hereinafter, operations 351 and 352 will be described in detail with reference to FIG. 6. In FIG. 6, 61 denotes first volume data, and 62 denotes second volume data. According to an embodiment, 61 denotes normal volume data, and 62 denotes abnormal volume data. The first volume data 61 and the second volume data 62 may include four pieces of sub volume data 61a, 61b, 61c, 61d and four pieces of sub volume data 62a, 62b, 62c and 62d, respectively.

First sub volume data obtained during a first ECG cycle t1 is determined as the first sub volume data 61a of the first volume data 61 since the first ECG cycle t1 is the first ECG cycle within the previously determined range. That is, the first volume data 61 is generated using the first sub volume data 61a obtained during the first ECG cycle t1. Thereafter, second sub volume data obtained during a second ECG cycle t2 is determined as the second sub volume data 61b of the first volume data 61 since the second ECG cycle t2 is the first ECG cycle within the previously determined range.

However, third sub volume data obtained during a third ECG cycle t3 is determined as the third sub volume data 62c of the second volume data 62 since the third ECG cycle t3 is the second ECG cycle that is outside and less than the previously determined range. The second ECG cycle outside the previously determined range may be an irregular abnormal ECG cycle due to arrhythmia that occurs in the target body 10. Thereafter, fourth sub volume data obtained during a fourth ECG cycle t4 is determined as the fourth sub volume data 62d of the second volume data 62 since the fourth ECG cycle t4 is the second ECG cycle.

As described above, a plurality of pieces of sub volume data obtained by sequentially scanning an N number of sub scan spaces may be determined as sub volume data of first volume data or second volume data based on each ECG cycle. Also, the first volume data or the second volume data may be generated using at least one piece of determined sub volume data.

Subsequent to the fourth ECG cycle t4, fifth, sixth, and seventh sub volume data obtained during fifth, sixth, and seventh ECG cycles t5, t6, and t7 have the first ECG cycle within the previously determined range, and thus the fifth, sixth, and seventh sub volume data are determined as the sub volume data 61a, 61b, 61c, 61d of the first volume data 61.

During this process, the first volume data 61 and the second volume data 62 include the four pieces of sub volume data 61a, 61b, 61c, 61d and the four pieces of sub volume data 62a, 62b, 62c and 62d, respectively, and thus the fifth sub volume data may replace the first sub volume data 61a and may be new first sub volume data.

That is, sub volume data newly obtained with respect to the same sub scan space includes image information more recently obtained than previously obtained sub volume data, and thus the newly obtained sub volume data may be used to generate first volume data or second volume data. In other words, the ultrasound diagnosis apparatus 100 may renew at least one of the first volume data and the second volume data using the newly obtained sub volume data.

Like the fifth sub volume data, the sixth sub volume data may replace the second sub volume data 61b of the first volume data 61 and may be new second sub volume data, and the seventh sub volume data may be new third volume data.

In operation 360, the ultrasound diagnosis apparatus 100 determines whether all sub volume data corresponding to the N number of sub scan spaces exist. That is, when the ultrasound diagnosis apparatus 100 performs operation 360 from operation 351, the ultrasound diagnosis apparatus 100 determines whether the first volume data includes all sub volume data corresponding to the plurality of sub scan spaces. When the ultrasound diagnosis apparatus 100 performs operation 360 from operation 352, the ultrasound diagnosis apparatus 100 determines whether the second volume data includes all sub volume data corresponding to the plurality of sub scan spaces. In other words, the ultrasound diagnosis apparatus 100 determines whether the first volume data or the second volume data is "complete" volume data.

Referring to FIG. 6, the first volume data 61 includes the first volume data 61a, the second volume data 61b, and the third volume data 61c. Thus, since the fourth sub volume data corresponding to the fourth sub scan space is not obtained, the first volume data 61 is "incomplete" volume data. Likewise, since the second volume data 62 includes the third volume data 62c and the fourth volume data 62d, the second volume data 62 is also incomplete volume data.

Although not shown in FIG. 6, in a case where eighth sub volume data is measured according to the first ECG cycle within the previously determined range during a subsequent ECG cycle t8, the ultrasound diagnosis apparatus 100 may determine the eighth sub volume data as the fourth sub volume data 61d of the first volume data 61. In this regard, the first volume data 61 may be complete volume data including all sub volume data corresponding to the four pieces of sub volume data 61a, 61b, 61c, and 61d. If the ultrasound diagnosis apparatus 100 determines that the first volume data or the second volume data is the complete volume data, the ultrasound diagnosis apparatus 100 performs operation 370. If the ultrasound diagnosis apparatus 100 determines that the first volume data or the second volume data is not the complete volume data, the ultrasound diagnosis apparatus 100 performs operation 380.

In operation 370, the ultrasound diagnosis apparatus 100 displays at least one piece of the first volume data and the second volume data. The ultrasound diagnosis apparatus 100 may display only the complete volume data or may display both the complete volume data and the incomplete volume data in operation 370. A detailed description of displaying volume data will be given with reference to FIG. 8. The displayed at least one piece of the first volume data and the second volume data may enable a user of the ultrasound diagnosis apparatus 100 to easily read diagnosis results of a normal ECG cycle and an abnormal ECG cycle.

If the ultrasound diagnosis apparatus 100 determines that the first volume data or the second volume data is not the complete volume data, in operation 380, the ultrasound diagnosis apparatus 100 determines whether i=N. That is, the ultrasound diagnosis apparatus 100 determines whether the N number of sub scan spaces is sequentially scanned.

If the ultrasound diagnosis apparatus 100 determines that i is not equal to N, the ultrasound diagnosis apparatus 100 performs operation 391. In other words, i increases by 1 and a next sub scan space is scanned. That i is not equal to N means that a 3D scan space is not wholly scanned, and thus a next sub scan space is scanned.

If the ultrasound diagnosis apparatus 100 determines that i is equal to N, the ultrasound diagnosis apparatus 100 performs operation 392. That is, i becomes 1 and a first sub scan space is scanned. That i is equal to N means that all sub scan spaces of the 3D scan space are wholly scanned. However, since the first volume data or the second volume data is not the complete volume data, a volume data generation process is repeatedly performed by scanning the 3D scan space again until the ultrasound diagnosis apparatus 100 determines that the first volume data or the second volume data is the complete volume data.

Referring to FIG. 6, after the fourth sub scan space is scanned (i=4), the first volume data or the second volume data is not the complete volume data. Thus, the ultrasound diagnosis apparatus 100 determines whether i=N, and, since i=N=4, the ultrasound diagnosis apparatus 100 determines that i=1 again. Thereafter, the first sub scan space is scanned again during the fifth ECG cycle t5.

The second and third sub scan spaces are sequentially scanned during the sixth and seventh ECG cycles t6 and t7. Although not shown, the fourth sub scan space is scanned during an eighth ECG cycle t8. If sub volume data obtained in the fourth sub scan space is determined as the sub volume data 61a, 61b, 61c, and 61d of the first volume data 61, the first volume data 61 is the complete volume data. Thereafter, at least one of the first volume data 61 and the second volume data 62 is displayed.

However, if the sub volume data obtained in the fourth sub scan space is determined as the sub volume data 62a, 62b, 62c, and 62d of the second volume data 62, since the first volume data 61 and the second volume data 62 are the incomplete volume data, the volume data generation process is repeatedly performed. That is, since i=4, the first sub scan space (i=1) is scanned again during a ninth ECG cycle t9. Such a repetitive process continues until the ultrasound diagnosis apparatus 100 determines that the first volume data or the second volume data is the complete volume data.

FIG. 7 shows a process of scanning a sub scan space selected by an external input signal and obtaining a plurality of pieces of sub volume data. First volume data 71 includes at least one piece of sub volume data obtained in a first ECG cycle and may be normal volume data. Second volume data 72 includes at least one piece of sub volume data obtained in a second ECG cycle and may be abnormal volume data.

An ECG cycle 701 in which first sub volume data is obtained is determined as a first ECG cycle, and thus the first sub volume data is determined as first sub volume data 71a of the first volume data 71. Thereafter, an ECG cycle 702 in which second sub volume data is obtained is determined as the first ECG cycle, and thus the second sub volume data is determined as second sub volume data 71b of the first volume data 71.

Thereafter, the external input signal used to select a third scan space "c" may be received during a process of obtaining third sub volume data in an ECG cycle 703. Third sub volume data obtained in the ECG cycle 703 determined as the first ECG cycle is determined as third sub volume data 71c of the first volume data 71 based on the external input signal.

Next, the external input signal used to select the third scan space "c" may be continuously received during a process of obtaining fourth sub volume data in an ECG cycle 704. In this regard, the fourth sub volume data obtained in the ECG cycle 704 determined as the second ECG cycle is determined as the third sub volume data 71c of the first volume data 71, other than fourth sub volume data 72d of the second volume data 72. That is, a sub scan space that is to be scanned may be determined among four sub scan spaces that are split from a 3D scan space based on the external input signal.

If the external input signal used to select the third scan space "c" is received in ECG cycles 705, 706, and 707, sub volume data obtained in the ECG cycles 705, 706, and 707 is determined as the third sub volume data 71c or third sub volume data 72c, irrespective of an order of previous sub volume data.

If the external input signal used to select the third scan space "c" is not received any more in an ECG cycle 708, a next sub scan space "d" is scanned. That is, a fourth sub scan space of the 3D scan space is scanned. Thus, eighth sub volume data obtained in the ECG cycle 708 is determined as fourth sub volume data 71d of the first volume data 71 since the ECG cycle 703 is determined as the first ECG cycle.

FIG. 8 shows an embodiment in which at least one of first volume data and second volume data is displayed. The ultrasound diagnosis apparatus 100 may display first volume data 810, a first ultrasound image 815, second volume data 820, and a second ultrasound image 825 on a screen 800 of a device. According to an embodiment, the first volume data 810 may be normal volume data, and the second volume data 820 may be abnormal volume data. The first ultrasound image 815 and the second ultrasound image 825 may be 2D or 3D images corresponding to each volume data.

According to an embodiment, the ultrasound diagnosis apparatus 100 may display complete volume data on the screen 800 of the device. That is, the ultrasound diagnosis apparatus 100 may display volume data including all sub volume data corresponding to a plurality of sub scan spaces, such as the first volume data 810.

In the present embodiment, the ultrasound diagnosis apparatus 100 may display only the first volume data 810 that is complete volume data and may not display the second volume data 820 that is incomplete volume data, whereas the ultrasound diagnosis apparatus 100 may display both the first volume data 810 that is the complete volume data and the second volume data 820 that is the incomplete volume data.

According to another embodiment, the ultrasound diagnosis apparatus 100 may change at least one of a color, chroma, and brightness of sub volume data that is not obtained from among a plurality of pieces of sub volume data to display the incomplete volume data.

That is, all sub volume data obtained through a third sub scan space may be used to generate the first volume data 810 by measuring an ECG cycle scanning the third sub scan space within a previously determined range from the second volume data 820. Thus, third sub scan volume data 821 of the second volume data 820 may not be obtained.

In this regard, according to an embodiment, the ultrasound diagnosis apparatus 100 may change at least one of a color, chroma, and brightness of a part corresponding to the third sub volume data 821 that is not obtained to distinguish and display the third sub volume data 821 from sub volume data that is obtained.

According to another embodiment, the ultrasound diagnosis apparatus 100 may extract, from the first volume data 810, and display the third sub scan volume data 821 of the second volume data 820 that is not obtained. That is, the third sub scan volume data 821 of the second volume data 820 may be third sub volume data of the first volume data 810. The second volume data 820 may be generated using at least one of the sub volume data included in the first volume data 810.

Regarding the second ultrasound image 825 corresponding to the second volume data 820 that is incomplete volume data, like the second volume data 820, sub volume data 826 that is not obtained may be distinguished and displayed from other sub volume data that is obtained.

The embodiments of the present invention may be written as computer programs and can be implemented in general-use digital computers that execute the programs using a computer readable recording medium. In addition, a structure of data used in the above-described method may be recorded in a computer readable recording medium through various methods. Examples of the computer readable recording medium include magnetic storage media (e.g., ROMs, RAMs, universal serial buses (USBs), floppy disks, hard disks, etc.), optical recording media (e.g., CD-ROMs, or DVDs), and storage media such as PC interfaces (e.g., PCI, PCI-express, WiFi, etc.).

A target body may be more efficiently and easily diagnosed by using the above-described ultrasound diagnosis apparatus and method using an ECG. That is, volume data is split into a plurality of sub volume data, thereby diagnosing the target body using the volume data having high resolution.

Also, the plurality of sub volume data is displayed after being classified into two pieces of volume data according to ECG cycles, thereby obtaining both volume data with respect to a normal ECG cycle and volume data with respect to an abnormal ECG cycle. A user of the ultrasound diagnosis apparatus separately observes the volume data according to the ECG cycles, thereby diagnosing the target body in more detail.

Furthermore, the user of the ultrasound diagnosis apparatus may sequentially obtain the plurality of sub volume data in real time while observing the target body and may obtain desired sub volume data only, thereby observing an optional region in more detail.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. An ultrasound diagnosis method using an electrocardiogram (ECG), the method comprising:
obtaining ECG information;
generating first volume data using at least one piece of sub volume data obtained in a first ECG cycle based on the ECG information;
generating second volume data using at least one piece of sub volume data obtained in a second ECG cycle based on the ECG information; and
displaying at least one of the first volume data and the second volume data.

2. The ultrasound diagnosis method of claim 1, further comprising:
determining an ECG cycle based on a time interval of ECG signals included in the ECG information.

3. The ultrasound diagnosis method of claim 2, wherein, in a case where the time interval of the ECG signals is within a previously determined range, the ECG cycle is determined as the first ECG cycle; and
in a case where the time interval of the ECG signals is outside the previously determined range, the ECG cycle is determined as the second ECG cycle.

4. The ultrasound diagnosis method of claim 3, wherein the first ECG cycle is a normal ECG cycle, and the second ECG cycle is an abnormal ECG cycle.

5. The ultrasound diagnosis method of claim 4, wherein the first volume data is normal volume data based on the normal ECG cycle, and the second volume data is abnormal volume data based on the abnormal ECG cycle.

6. The ultrasound diagnosis method of claim 1, further comprising:
splitting a 3D scan space into a plurality of sub scan spaces; and
obtaining a plurality of pieces of sub volume data corresponding to the plurality of respective sub scan spaces by sequentially scanning the plurality of sub scan spaces according to the ECG cycles included in the ECG information.

7. The ultrasound diagnosis method of claim 1, further comprising:
splitting a 3D scan space into a plurality of sub scan spaces; and
obtaining a plurality of pieces of sub volume data corresponding to the sub scan spaces selected from the plurality of sub scan spaces based on an external input signal.

8. The ultrasound diagnosis method of claim 1, further comprising:
splitting a 3D scan space into a plurality of sub scan spaces,
wherein the displaying comprises: displaying volume data including all of a plurality of pieces of sub volume data corresponding to the plurality of respective sub scan spaces.

9. The ultrasound diagnosis method of claim 1, wherein the displaying comprises: displaying at least one of the at least one sub volume data used to generate the first volume data and the at least one sub volume data used to generate the second volume data and an identification mark.

10. The ultrasound diagnosis method of claim 5, wherein the abnormal ECG cycle comprises at least one of an atrial flutter ECG cycle, an atrial fibrillation ECG cycle, a ventricular flutter ECG cycle, and a ventricular fibrillation ECG cycle.

11. An ultrasound diagnosis apparatus using an ECG, the apparatus comprising:
an ECG information obtaining unit for obtaining ECG information;
a volume data processing unit for generating first volume data using at least one piece of sub volume data obtained in a first ECG cycle based on the ECG information, and generating second volume data using at least one piece of sub volume data obtained in a second ECG cycle based on the ECG information; and
a display unit for displaying at least one of the first volume data and the second volume data.

12. The ultrasound diagnosis apparatus of claim 11, wherein the ECG information obtaining unit determines an ECG cycle based on a time interval of ECG signals included in the ECG information.

13. The ultrasound diagnosis apparatus of claim 12, wherein, in a case where the time interval of the ECG signals is within a previously determined range, the ECG information obtaining unit determines the ECG cycle as the first ECG cycle, and, in a case where the time interval of the ECG signals is outside the previously determined range, determines the ECG cycle as the second ECG cycle.

14. The ultrasound diagnosis apparatus of claim 13, wherein the first ECG cycle is a normal ECG cycle, and the second ECG cycle is an abnormal ECG cycle.

15. A computer readable recording medium storing a program for executing an ultrasound diagnosis method of claim 1.
